# EUROPÄISCHE PATENTSCHRIFT

(11) **EP 2 452 642 B1**
(45) Veröffentlichungstag und Bekanntmachung des Hinweises auf die Patenterteilung: **03.08.2016**
(21) Anmeldenummer: 11185949.2
(22) Anmeldetag: 20.10.2011
(51) Int. Cl.: A61B 17/70

(54) **Pedikelschraube**
Pedicle screw
Vis pédiculaire

(30) Priorität: 15.11.2010 DE 102010060555
(43) Veröffentlichungstag der Anmeldung: 16.05.2012
(73) Patentinhaber: Ulrich GmbH & Co. KG, 89081 Ulm (DE)
(72) Erfinder: Werner, Claudia, 89233 Neu- Ulm (DE); Midderhoff, Stefan, 89075 Ulm (DE)
(74) Vertreter: Hentrich Patentanwälte PartG mbB

(56) Entgegenhaltungen:
- US-A1- 2008 154 315
- US-A1- 2009 062 861
- US-A1- 2009 076 552
- US-A1- 2010 211 114

## Beschreibung

Die Erfindung betrifft eine Pedikelschraube für Implantate zur Korrektur und Stabilisierung der Wirbelsäule, mit einem an dem einen axialen Ende eines Gewindeschaftes angeordneten Kopfteil und einem Tulpenkopf mit einer in Richtung des Gewindeschaftes offenen Aufnahme für das Kopfteil, sowie einer zwischen den Schenkeln des Tulpenkopfes ausgebildete Stabaufnahme zur Fixierung eines Stabes, wobei in der Aufnahme ein das Kopfteil von unten, nämlich aus Richtung des Gewindeschaftes haltender Kopfteilhalter angeordnet und schwenkbar gelagert ist.

Eine derartige Pedikelschraube ist aus der US 2008/0154315 A1 bekannt, bei der der Kopfteilhalter als einseitig offener Sicherungsring gebildet ist, der mittels Federwirkung in den Kopfteil eingesetzt werden kann.

Die US 2009/0076552 A1 zeigt eine Pedikelschraube, bei der das Kopfteil zweiteilig ausgeführt ist mit einem Rundkopf und einem Lager. Die Sicherung des Kopfteils erfolgt mittels eines Stiftes, der die fluchtenden Bohrungen des Lagers, des Kopfteils und des Tulpenkopfes durchsetzt.

Eine weitere Pedikelschraube ist aus der EP 2 078 506 A1 bekannt. Sie zeigt einen Gewindeschaft, welcher sich polyaxial bezüglich der Tulpenkopflängsachse um maximal 45 Grad auslenken lässt, und sie weist eine Aufnahme des Kopfteils auf, welche aus flexiblen Streifen gebildet ist, die über die Äquatoriallinie des kugelförmigen Kopfteils greifen. Dadurch wird das kugelförmige Kopfteil mittels Klemmung konzentrisch zur Tulpenkopflängsachse im Tulpenkopf gehalten.

In der WO 2010/065648 ist eine entsprechende Pedikelschraube gezeigt, welche auch hier einen Schnappverschluss zur Halterung des kugelförmigen Kopfteils vorsieht. Die Finger greifen über die Äquatoriallinie des kugelförmigen Kopfteils hinaus, wodurch ein um maximal 45 Grad polyaxiales Auslenken des Gewindeschaftes gegenüber der Tulpenkopflängsachse ermöglicht ist.

Die US 2005/0 203 516 A1 zeigt eine Pedikelschraube, die einen im Querschnitt rechteckig geformten Stab aufnehmen kann. In einer Ausführungsform ist der Gewindeschaft vom Kopfteil abtrennbar, wobei das Kopfteil im Gewindeschaft radial zu der Wirkenkopflängsachse gelagert ist. Durch ein Langloch lässt sich der Gewindeschaft bezüglich des Kopfteils um die Tulpenkopflängsachse verschwenken. Dieses relative Verdrehen soll die Schraube bei Festziehen des Stabes davor bewahren aus dem Knochen der Wirbelsäule herausgedreht zu werden. Auch hier ist keine Auslenkung des Gewindeschaftes von mehr als 45 Grad ermöglicht.

Um eine spinale Fixation sinnvoll durchführen zu können ist es notwendig, dass der Gewindeschaft gegenüber der Tulpenkopflängsachse polyaxial auslenkbar ist, um den später wirbelknochenverbindenden Stab fest und sicher an der Pedikelschraube anbringen zu können. Der Gewindeschaft der Pedikelschraube wird üblicherweise an den massiven Stellen des Knochens eingedreht, wodurch aufwendige Maßnahmen zum Knochenaufbau vermieden werden. Die Gewindeschäfte der verwendeten Pedikelschrauben werden deshalb häufig an unterschiedlicher Stelle oder unter unterschiedlichem Winkel in die Knochen eingedreht. Ein beweglicher Tulpenkopf ist dabei sehr hilfreich, um den die Knochen verbindenden Stab fest und sicher an dem Kopfteil der Pedikelschraube zu fixieren.

Es ist daher die Aufgabe der Erfindung eine Pedikelschraube der eingangs genannter Art bereitzustellen, bei der der Gewindeschaft bezüglich der Tulpenkopflängsachse weiter auslenkbar ist als bei den bisher bekannten Pedikelschrauben.

Diese Aufgabe wird mit der Erfindung dadurch gelöst, dass der Kopfteilhalter schwenkbar gelagert ist, um eine radial zur Tulpenkopflängsachse orientierte Achse über zwei Stiftaufnahmen mittels zweier in Bohrungen des Tulpenkapfes eingeführte Stifte.

Diese Ausführungsform der Pedikelschraube bietet den Vorteil, dass das Kopfteil im Kopfteilhalter um maximal 45 Grad bezüglich der Tulpenkopflängsachse ausgelenkt werden kann, und dass eine weitere Auslenkung durch die schwenkbare Lagerung des Kopfteilhalters ermöglicht ist.

Es ist eine sichere Lagerung des Kopfteilhalters im Tulpenkopf gewährleistet und somit ein Herausfallen des Kopfteilhalters aus dem Tulpenkopf unterbunden. Die Stiftaufnahmen dienen folglich der sicheren Lagerung des Kopfteilhalters.

Eine weitere vorteilhafte Ausführungsform ist dadurch gegeben, dass die Stiftaufnahmen in Richtung des Gewindeschaftes offen sind. Somit kann der Kopfteilhalter bei der hierbei erleichterten Montage der Pedikelschraube von oben in den Tulpenkopf eingeführt werden, um die Stiftaufnahmen mit den Stiften lösbar zu verbinden.

Weiterhin ist von Vorteil, wenn der Kopfteilhalter senkrecht zu einer durch die zwei Stifte gebildeten Lagerachse eine im Querschnitt V-förmige Krümmung mit Öffnung in Richtung des Gewindeschaftes aufweist. Hierdurch kann der Kopfteilhalter das kugelförmige Kopfteil schalenartig von unten im Tulpenkopf halten. Während die Halter der EP 2 078 506 A1, der WO 2010/065 648 und der US 2005/0 203 516 A1 das Kopfteil von der dem Gewindeschaft abgewandten Seite umgreifen, unterbindet die Halterung von unten, nämlich von der dem Gewindeschaft zugewandten Seite, dass sich das Kopfteil und der Tulpenkopf aufgrund von wirkenden Zugkräften voneinander lösen.

Ein weiterer Vorteil ist dadurch gegeben, dass der Gewindeschaft polyaxial relativ zur Tulpenkopflängsachse auslenkbar ist. Dadurch ist gewährleistet, dass sich der Gewindeschaft nicht nur entlang der Richtung der Schwenkbewegung auslenken lässt, sondern auch in jeder anderen beliebigen Richtung. Dadurch ist wiederum eine erleichterte Aufnahme des Stabes im Tulpenkopf ermöglicht.

Ein weiterer Vorteil ist damit verbunden, dass der Tulpenkopf an seinem dem Kopfteil zugewandten Ende mindestens eine Aussparung zur Aufnahme des Gewindeschafts aufweist. Hierdurch ist eine erweiterte Auslenkung des Gewindeschafts bezüglich der Tulpenkopflängsachse ermöglicht, da dieser durch die Aussparung erst später in seiner Auslenkung blockiert wird. Die Geometrie des Tulpenkopfes und des Kopfteilhalters sind demnach die begrenzenden Elemente zur Auslenkung des Gewindeschaftes relativ zur Tulpenkopflängsachse. Ist die Aussparung senkrecht zur Lagerachse angeordnet, so kann der Gewindeschaft zunächst durch die Schwenkbewegung des Kopfteilhalters ausgelenkt werden, anschließend durch die Gleitbewegung des Kopfteils im Kopfteilhalter, wodurch eine erhöhte Auslenkung des Gewindeschaftes bezüglich der Kopfteillängsachse ermöglicht ist.

Vorteilhaft ist auch, wenn der Gewindeschaft in einer ersten Richtung relativ zur Tulpenkopflängsachse durch eine schwenkende Bewegung des Kopfteilhalters und eine gleitende Bewegung des Kopfteils im Kopfteilhalter um einen größeren Winkel auslenkbar ist, als in einer zweiten Richtung durch eine gleitende Bewegung des Kopfteils im Kopfteilhalter. Dadurch kann der Tulpenkopf derart am Kopfteil angebracht werden, dass dieser in einer ersten Richtung weiter verkippbar ist, um den stabilisierenden Stab ordnungsgemäß in der Stabaufnahme lagern zu können, wenn der Gewindeschaft beispielsweise besonders steil, nämlich unter großem Winkel zur Normalen der Fläche, in der die Schraube fixiert werden soll im Knochen verankert ist.

Ist die erste Richtung senkrecht zur Lagerachse des Kopfteilhalters angeordnet, so ist damit der Vorteil verbunden, dass auch hier die Schwenkbewegung des Kopfteilhalters und die Gleitbewegung des Kopfteils im Kopfteilhalter in der ersten Richtung bezüglich der beiden Auslenkwinkel ausführbar ist. Dabei ist von Vorteil, wenn der Gewindeschaft in der ersten Richtung um maximal 61 Grad und in der zweiten Richtung um maximal 45 Grad auslenkbar ist. Hieraus folgen Abmessungen für den Gewindeschaft, den Kopfteilhalter und den Tulpenkopf, welche eine sehr hohe Stabilität aufweisen und zusätzlich hinreichend klein gebildet sind, dass diese nicht störend auf den Patienten wirken.

Eine weitere vorteilhafte Ausführungsform besteht darin, dass der Tulpenkopf radial zur Tulpenkopflängsachse Bohrungen aufweist. Diese Bohrungen erleichtern die Montage der Stifte zur schwenkbaren Lagerung des Kopfteilhalters im Tulpenkopf. Die Stifte lassen sich so leicht radial zur Tulpenkopflängsachse von außen in den Tulpenkopf einschieben. Außerdem können über die Bohrung weitere Komponenten im Tulpenkopf gesichert werden, wobei von Vorteil ist, dass ein Klemmteil mittels mindestens einem Sicherungsstift im Tulpenkopf sicherbar ist. Dieses Klemmteil bewirkt, dass der Tulpenkopf nicht über den Gewindeschaft rutschen kann. Außerdem dient er der Führung des kugelförmigen Kopfteils im Tulpenkopf. Weist das Klemmteil eine Werkzeugführung auf, so ist damit der Vorteil verbunden dass der Gewindeschaft über das Kopfteil und durch die Werkzeugführung mit einem Werkzeug im Knochen verschraubt und mit diesem verankert werden kann.

Weiterhin ist von Vorteil, dass am Tulpenkopf im axialen Abstand von dem dem Stab zugewandten Ende mindestens eine Führungsnut angeordnet ist. Hierdurch kann weiteres Werkzeug, wie zum Beispiel eine perkutane Hülse, am Tulpenkopf befestigt werden, um diesen zur Aufnahme des Stabes auszurichten.

Von Vorteil ist auch, wenn der Tulpenkopf in seiner axialen Erstreckung mit sich verjüngendem Außendurchmesser gebildet ist.

Zur Fixierung des Stabes in der Stabaufnahme ist von Vorteil, wenn am Innenumfang des dem Stab zugewandten Endes der Schenkel ein Spindelgewinde angeordnet ist. Dadurch kann eine Schraube in den Tulpenkopf eingedreht werden, die den Stab im Tulpenkopf am Kopfteil fixiert.

Im Folgenden wird die Erfindung an in der Zeichnung dargestellten Ausführungsbeispielen näher erläutert; es zeigen:
- Fig. 1: eine Seitenansicht der Redikelschraube bezüglich der ersten Richtung,
- Fig. 2: einen Längsschnitt der Pedikelschraube bezüglich der ersten Richtung,
- Fig. 3: eine Explosionsansicht der Pedikelschraube mit Ansicht der ersten Richtung,
- Fig. 4: eine Explosionsansicht der Pedikelschraube mit Ansicht der zweiten Richtung,
- Fig. 5a: eine Seiteneinsicht der Pedikelschraube mit in der ersten Richtung ausgelenktem Gewindeschaft,
- Fig. 5b: einen.Längsschnitt der Pedikelschraube mit in der ersten Richtung ausgelenktem Gewindeschaft,
- Fig. 6a: eine Seitenansicht der Pedikelschraube mit in der zweiten Richtung ausgelenktem Gewindeschaft,
- Fig. 6b: einen Längsschnitt der Pedikelschraube mit in der zweiten Richtung ausgelenktem Gewindeschaft, und
- Fig. 7.: eine um 90 Grad entlang der Tulpenkopflängsachse gedrehte Darstellung von Figur 6a.

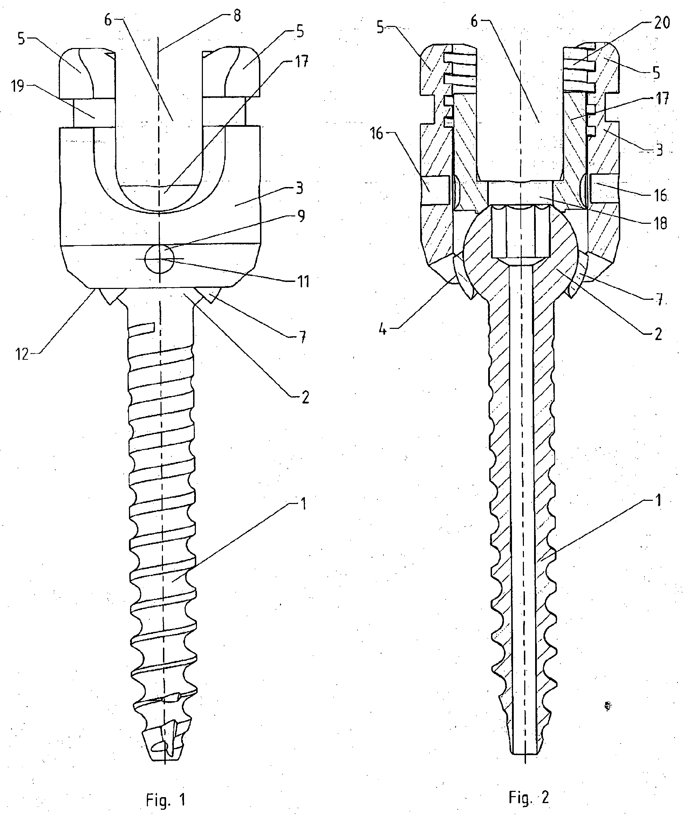

In Figur 1 ist die erfindungsgemäße Pedikelschraube gezeigt, wobei hier der Gewindeschaft 1 bezüglich der Tulpenkopflängsachse 8 nicht ausgelenkt ist. Der Kopfteilhalter 7 ist über die Stifte 9 radial zur Tulpenkopflängsachse 8 im Tulpenkopf 3 gelagert. Der Kopfteilhalter 7 weist hierbei senkrecht zu einer durch die zwei Stifte 9 gebildeten Lagerachse 11 eine im Querschnitt V-förmige Krümmung mit Öffnung in Richtung des Gewindeschaftes 1 auf. Im Tulpenkopf 3 ist desweiteren ein Klemmteil 17 angeordnet.

Figur 2 zeigt eine entlang der Tulpenkopflängsachse 8 geschnittene Ansicht der Pedikelschraube, wobei hier ersichtlich ist, dass das Kopfteil 2 vom Kopfteilhalter 7 von unten, nämlich aus Richtung des Gewindeschaftes 1 im Tulpenkopf 3 gehalten ist. Das Klemmteil 17 zeigt eine Werkzeugführung 18 und das Kopfteil 2 eine Aufnahme für ein Werkzeug. Außerdem sieht der Gewindeschaft 1 radiale Bohrungen in seiner Wandung vor und ein Kanal zum Einfüllen von verfestigenden Materialien ist dargestellt. Das Klemmteil 17 ist hier über Bohrungen 16 im Tulpenkopf 3 gesichert, wobei der Tulpenkopf 3 an der Innenwandung ein Spindelgewinde 20 aufweist zur Sicherung des später einzuführenden Stabes. Im axialen Abstand von dem dem Stab zugewandten Ende ist eine Führungsnut 19 angeordnet.

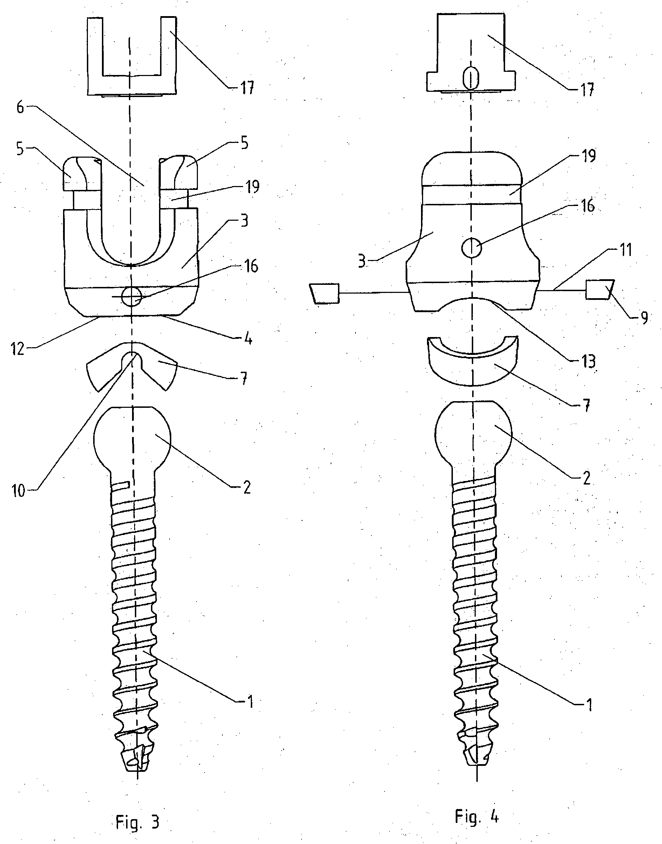

In Figur 3 ist eine Explosionsansicht entlang der Tulpenkopflängsachse 8 der Pedikelschraube dargestellt, wobei hier die Bohrung 16 zur Stifteinführung im Tulpenkopf 3 gezeigt ist. Der Kopfteilhalter 7 weist hierbei zwei Stiftaufnahmen 10 auf und seine V-förmige Krümmung ist deutlich erkennbar.

Figur 4 zeigt eine entlang der Tulpenkopflängsachse 8 um 90 Grad gedrehte Darstellung der Figur 3, wobei hier der Tulpenkopf 3 in seiner axialen Erstreckung mit sich verjüngendem Außendurchmesser gebildet ist. Desweiteren erkennt man hier die Aussparung 13 zur Aufnahme des Gewindeschaftes 1.

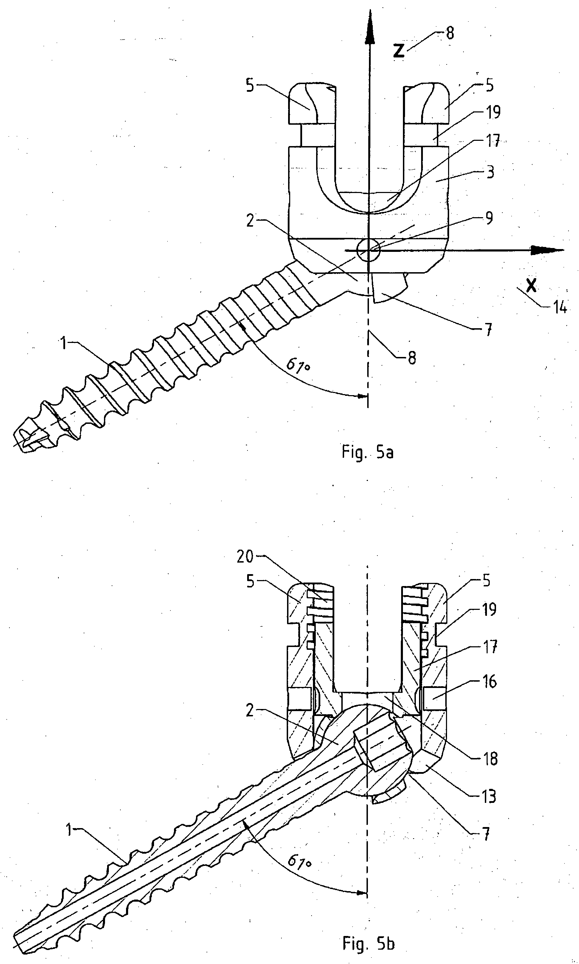

Die Figuren 5a und 5b zeigen die Auslenkung des Gewindeschaftes 1 bezüglich der Tulpenkopflängsachse 8 in der ersten Richtung 14. Hierbei wird der Kopfteilhalter 7 entlang der Lagerachse 11 mittels einer Schwenkbewegung ausgelenkt, bis er am Klemmteil 17 im Tulpenkopf 3 anstößt. Ebenso könnte er an Vorsprüngen oder Ausprägungen, die der Tulpenkopf 3 selbst vorsieht, anstoßen. Nachdem sich der Kopfteilhalter 7 nicht mehr weiter auslenken lässt, kann nunmehr das Kopfteil 2 im Kopfteilhalter 7 gleiten, wodurch eine erweiterte Auslenkung möglich ist. In der Zeichnung stößt der Gewindeschaft 1 am Kopfteilhalter 7 oder am Ende der Aussparung 13 des Tulpenkopfes 3 an. Die Zeichnung zeigt vorteilhafte Abmessungen der Komponenten der Pedikelschraube wodurch eine Auslenkung in der ersten Richtung 14 um 61 Grad, wie in der Zeichnung dargestellt, erreichbar ist.

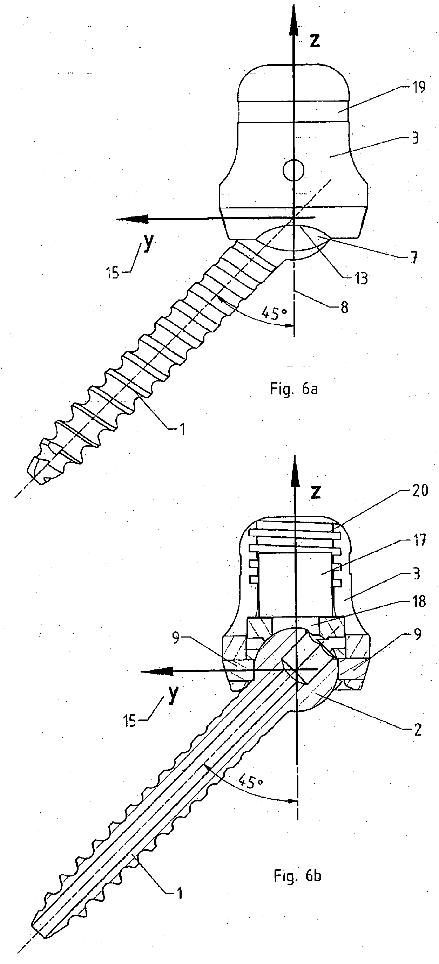

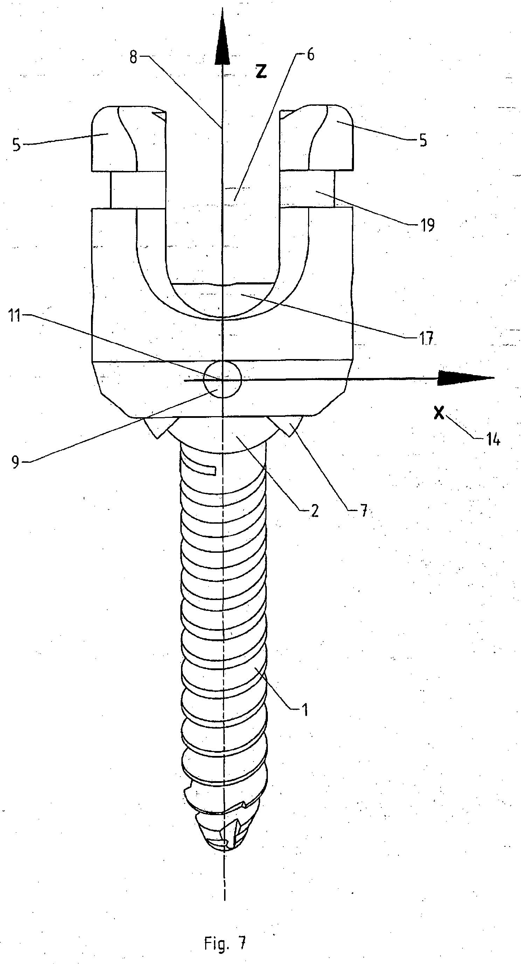

Die Figuren 6a, 6b und 7 zeigen die Pedikelschraube mit Auslenkung des Gewindeschafts 1 bezüglich der Tulpenkopflängsachse 8 in der zweiten Richtung 15. Hierbei ist keine Auslenkung oder Schwenkung des Kopfteilhalters 7 in der zweiten Richtung 15 möglich. Lediglich die Gleitbewegung des Kopfteils 2 im Kopfteilhalter 7 bewirkt die Auslenkung des Gewindeschafts 1 bezüglich der Tulpenkopflängsachse 8.

Die Zeichnung zeigt die Ausführungsform mit den vorteilhaften Abmessungen der einzelnen Komponenten der Pedikelschraube, wodurch ein Auslenkwinkel in der zweiten Richtung nach Figur 6a von 45 Grad erreichbar ist.

Die Figur 7 zeigt eine entlang der Tulpenkopflängsachse 8 um 90 Grad gedrehte Darstellung der Figuren 6a und 6b, wobei hier nochmals gezeigt ist, dass die Auslenkung des Gewindeschaftes 1 parallel zur Lagerachse 11 geschieht.

### Bezugszeichenliste

- 1: Gewindeschaft
- 2: Kopfteil
- 3: Tulpenkopf
- 4: Aufnahme
- 5: Schenkel
- 6: Stabaufnahme
- 7: Köpfteilhalter
- 8: Tulpenkopflängsachse
- 9: Stift
- 10: Stiftaufnahme
- 11: Lagerachse
- 12: Kopfteil zugewandtes Ende
- 13: Aussparung
- 14: erste Richtung
- 15: zweite Richtung
- 16: Bohrungen
- 17: Klemmteil
- 18: Werkzeugführung
- 19: Führungsnut
- 20: Spindelgewinde

## Patentansprüche

1. Pedikelschraube für Implantate zur Korrektur und Stabilisierung der Wirbelsäule, mit einem an dem einen axialen Ende eines Gewindeschaftes (1) angeordneten Kopfteil (2) und einem Tulpenkopf (3) mit einer in Richtung des Gewindeschaftes (1) offenen Aufnahme (4) für das Kopfteil (2), sowie einer zwischen den Schenkeln (5) des Tulpenkopfes (3) ausgebildeten Stabaufnahme (6) zur Fixierung eines Stabes, wobei in der Aufnahme (4) ein das Kopfteil (2) von unten, nämlich aus Richtung des Gewindeschaftes (1) haltender Kopfteilhalter (7) angeordnet und schwenkbar gelagert ist, **dadurch gekennzeichnet, dass** der Kopfteilhalter (7) schwenkbar gelagert ist, um eine radial zur Tulpenkopflängsachse (8) orientierte Achse über zwei Stiftaufnahmen (10) mittels zweier in Bohrungen (16) des Tulpenkopfes (3) eingeführter Stifte (9).

2. Pedikelschraube nach Anspruch 1, **dadurch gekennzeichnet, dass** die Stiftaufnahmen (10) in Richtung des Gewindeschaftes (1) offen sind.

3. Pedikelschraube nach Anspruch 1 oder 2, **dadurch gekennzeichnet, dass** der Kopfteilhalter (7) senkrecht zu einer durch die zwei Stifte gebildeten Lagerachse (11) eine im Querschnitt V-förmige Krümmung mit Öffnung in Richtung des Gewindeschaftes (1) aufweist.

4. Pedikelschraube nach einem der Ansprüche 1 bis 3, **dadurch gekennzeichnet, dass** der Gewindeschaft (1) polyaxial relativ zur Tulpenkopflängsachse (8) auslenkbar ist.

5. Pedikelschraube nach einem der Ansprüche 1 bis3, **dadurch gekennzeichnet, dass** der Tulpenkopf (3) an seinen dem Kopfteil (2) zugewandten Ende (12) mindestens eine Aussparung (13) zur Aufnahme des Gewindeschafts (1) aufweist.

6. Pedikelschraube nach Anspruch 5, **dadurch gekennzeichnet, dass** die mindestens eine Aussparung (13) senkrecht zur Lagerachse (11) angeordnet ist.

7. Pedikelschraube nach einem der Ansprüche 1 bis 5, **dadurch gekennzeichnet, dass** der Gewindeschaft (1) in einer ersten Richtung (14) relativ zur Tulpenkopflängsachse (8) durch eine schwenkende Bewegung des Kopfteilhalters (7) und eine gleitende Bewegung des Kopfteils (2) im Kopfteilhalter (7) um einen größeren Winkel auslenkbar ist als in einer zweiten Richtung (15) durch eine gleitende Bewegung des Kopfteils (2) im Kopfteilhalter (15).

8. Pedikelschraube nach Anspruch 7, **dadurch gekennzeichnet, dass** die erste Richtung (14) senkrecht zur Lagerachse (11) des Kopfteilhalters (7) liegt.

9. Pedikelschraube nach Anspruch 7 oder 8, **dadurch gekennzeichnet, dass** der Gewindeschaft (1) in der ersten Richtung (14) um maximal 61 Grad und in der zweiten Richtung (15) um maximal 45 Grad auslenkbar ist.

10. Pedikelschraube nach einem der Ansprüche 1 bis 9, **dadurch gekennzeichnet, dass** ein Klemmteil (17) mittels mindestens einem Sicherungsstift im Tulpenkopf (3) sicherbar ist.

11. Pedikelschraube nach Anspruch 10, **dadurch gekennzeichnet, dass** das Klemmteil (17) eine Werkzeugführung (18) aufweist.

12. Pedikelschrauben nach Anspruch 10 oder 11, **dadurch gekennzeichnet, dass** am Tulpenkopf (3) in axialem Abstand von dem dem Stab zugewandten Ende der Schenkel (5) mindestens eine Führungsnut (19) angeordnet ist.

13. Pedikelschraube nach einem der Ansprüche 1 bis 12, **dadurch gekennzeichnet, dass** der Tulpenkopf (3) in seiner axialen Erstreckung mit sich verjüngendem Außendurchmesser gebildet ist.

14. Pedikelschraube nach einem der Ansprüche 1 bis 13, **dadurch gekennzeichnet, dass** am Innenumfang des dem Stab zugewandten Endes der Schenkel (5) ein Spindelgewinde (20) angeordnet ist.

## Claims

1. A pedicle screw for implants for correcting and stabilizing the spinal cord, having a head part (2) at one axial end of a screw shaft (1) and a tulip head (3) having a seat (4) that is open toward the screw shaft (1) for the head part (2), and a rod seat (6) between the flanks (5) of the tulip head (3) for the fixation of a rod, wherein a head retainer (7) located in the seat (4) retains the head part (2) from below, namely from the direction of the screw shaft (1), and is pivotable, **characterized in that** the head retainer (7) is pivotable around an axis oriented radial to the longitudinal tulip head axis (8) in two pin mountings (10) via two pins (9) inserted into bores (16) of the tulip head (3).

2. The pedicle screw according to claim 1, **characterized in that** the pin mountings (10) are open toward the screw shaft (1).

3. The pedicle screw according to claim 1 or 2, **characterized in that** the head retainer (7) has perpendicular to a bearing axis (11) formed by the two pins a bending V-shaped in cross section with opening towards the screw shaft (1).

4. The pedicle screw according to any of claims 1 to 3, **characterized in that** the screw shaft (1) can be deflected polyaxially relative to the longitudinal tulip head axis (8).

5. The pedicle screw according to any of claims 1 to 3, **characterized in that** the tulip head (3) has at its end (12) facing towards the head part (2) at least one cutout (13) for receiving the screw shaft (1).

6. The pedicle screw according to claim 5, **characterized in that** the at least one cutout (13) is aligned perpendicular to the bearing axis (11).

7. The pedicle screw according to any of claims 1 to 5, **characterized in that** the screw shaft (1) can be deflected in a first direction (14) relative to the longitudinal tulip head axis (8) by a pivoting motion of the head retainer (7) and a sliding motion of the head part (2) in the head retainer (7) by a larger angle than in a second direction (15) by a sliding motion of the head part (2) in the head retainer (15).

8. The pedicle screw according to claim 7, **characterized in that** the first direction (14) extends perpendicular to the bearing axis (11) of the head retainer (7).

9. The pedicle screw according to claim 7 or 8, **characterized in that**, the screw shaft (1) can be deflected in the first direction (14) by a maximum of 61 degree, and in the second direction (15) by a maximum of 45 degree.

10. The pedicle screw according to any of claims 1 to 9, **characterized in that** a clamping piece (17) is securable in the tulip head (3) by using at least one locking pin.

11. The pedicle screw according to claim 10, **characterized in that** the clamping piece (17) has a tool guide (18).

12. The pedicle screw according to claim 10 or 11, **characterized in that** the tulip head (3) has at least one guide groove (19) axially spaced from the end of the flanks (5) facing towards the rod.

13. The pedicle screw according to any of claims 1 to 12, **characterized in that** the tulip head (3) is designed with an axially decreasing outer diameter.

14. The pedicle screw according to any of claims 1 to 13, **characterized in that** the inner surface of the end of the flanks (5) facing towards the rod is provided with a spindle thread (20).

## Revendications

1. Vis pédiculaire pour implants destinés à la correction et à la stabilisation de la colonne vertébrale, comprenant une partie de tête (2), disposée sur l'une des extrémités axiales d'une tige filetée (1), et une tête tulipe (3), dotée d'un logement (4) ouvert en direction de la tige filetée (1) et destiné à la partie de tête (2), ainsi qu'un logement de tige (6) réalisé entre les branches (5) de la tête tulipe (3) et destiné à la fixation d'une tige, sachant qu'un support de partie de tête (7), qui tient la partie de tête (2) d'en bas, c'est-à-dire à partir de la tige filetée (1), est disposé, et monté avec possibilité de pivotement, dans le logement (4), **caractérisée en ce que** le support de partie de tête (7) est monté avec possibilité de pivotement autour d'un axe orienté radialement par rapport à l'axe longitudinal de tête tulipe (8), par l'intermédiaire de deux logements de broche (10), au moyen de deux broches (9) introduites dans des trous (16) de la tête tulipe (3).

2. Vis pédiculaire selon la revendication 1, **caractérisée en ce que** les logements de broche (10) sont ouverts en direction de la tige filetée (1).

3. Vis pédiculaire selon la revendication 1 ou 2, **caractérisée en ce que**, perpendiculairement à un axe de support (11) formé par les deux broches, le support de partie de tête (7) présente une courbure à section en forme de V, avec ouverture en direction de la tige filetée (1).

4. Vis pédiculaire selon l'une des revendications 1 à 3, **caractérisée en ce que** la tige filetée (1) peut être déviée de façon polyaxiale par rapport à l'axe longitudinal de tête tulipe (8).

5. Vis pédiculaire selon l'une des revendications 1 à 3, **caractérisée en ce que** la tête tulipe (3) présente, à son extrémité (12) tournée vers la partie de tête (2), au moins un évidement (13) destiné à recevoir la tige filetée (1).

6. Vis pédiculaire selon la revendication 5, **caractérisée en ce que** l'évidement (13), au nombre d'au moins un, est disposé perpendiculairement l'axe de support (11).

7. Vis pédiculaire selon l'une des revendications 1 à 5, **caractérisée en ce que** la tige filetée (1) peut être déviée dans une première direction (14) par rapport à l'axe longitudinal de tête tulipe (8), par un mouvement pivotant du support de partie de tête (7) et un mouvement coulissant de la partie de tête (2) dans le support de partie de tête (7), sous un angle qui est plus grand que dans une deuxième direction (15), par un mouvement coulissant de la partie de tête (2) dans le support de partie de tête (15).

8. Vis pédiculaire selon la revendication 7, **caractérisée en ce que** la première direction (14) est perpendiculaire à l'axe longitudinal (11) du support de partie de tête (7).

9. Vis pédiculaire selon la revendication 7 ou 8, **caractérisée en ce que** la tige filetée (1) peut être déviée d'au maximum 61 degrés dans 1a première direction (14) et d'au maximum 45 degrés dans la deuxième direction (15).

10. Vis pédiculaire selon l'une des revendications 1 à 9, **caractérisée en ce qu'**un élément de serrage (17) peut être retenu dans la tête tulipe (3) au moyen d'au moins une broche d'arrêt.

11. Vis pédiculaire selon la revendication 10, **caractérisée en ce que** l'élément de serrage (17) présente un guide d'outil (18).

12. Vis pédiculaire selon la revendication 10 ou 11, **caractérisée en ce qu'**au moins une rainure de guidage (19) est disposée sur la tête tulipe (3), à distance axiale de l'extrémité des branches (5) qui est tournée vers la tige.

13. Vis pédiculaire selon l'une des revendications 1 à 12, **caractérisée en ce que** la tête tulipe (3) est réalisée avec un diamètre extérieur diminuant dans sa dimension axiale.

14. Vis pédiculaire selon l'une des revendications 1 à 13, **caractérisée en ce qu'**un filetage hélicoïdal (20) est disposé sur la périphérie intérieure de l'extrémité des branches (5) qui est tournée vers la tige.
